(19) Europäisches Patentamt
European Patent Office
Office européen des brevets

(11) **EP 1 316 302 B1**

(12) **FASCICULE DE BREVET EUROPEEN**

(45) Date de publication et mention
de la délivrance du brevet:
**05.09.2007 Bulletin 2007/36**

(51) Int Cl.:
*A61K 8/67* (2006.01)     *A61K 8/81* (2006.01)
*A61Q 5/00* (2006.01)     *A61Q 19/00* (2006.01)

(21) Numéro de dépôt: **02292808.9**

(22) Date de dépôt: **12.11.2002**

(54) **Composition cosmétique ou dermatologique contenant de l'acide ascorbique stabilisé par au moins un polymère ou copolymère de N-vinylimidazole**

Kosmetische und/oder dermatologische Zusammensetzung, die durch mindestens ein Polymer oder Copolymer aus N-Vinylimidazol stabilisierte Ascorbinsäure enthält

Cosmetic and/or dermatological composition containing ascorbic acid stabilised by at least one polymer or copolymer of N-vinylimidazole

(84) Etats contractants désignés:
**AT BE BG CH CY CZ DE DK EE ES FI FR GB GR IE IT LI LU MC NL PT SE SK TR**

(30) Priorité: **28.11.2001 FR 0115375**

(43) Date de publication de la demande:
**04.06.2003 Bulletin 2003/23**

(73) Titulaire: **L'ORÉAL**
**75008 Paris (FR)**

(72) Inventeurs:
• **Biatry, Bruno**
**94300 Vincennes (FR)**

• **Lheureux, Eric**
**91230 Montgeron (FR)**

(74) Mandataire: **Kromer, Christophe**
**L'Oreal - D.I.P.I.**
**25-29 Quai Aulagnier**
**92665 Asnieres-Sur -Seine Cedex (FR)**

(56) Documents cités:
**EP-A- 0 884 047**     **EP-A- 1 064 924**
**WO-A-00/30594**     **WO-A-02/14876**
**US-B1- 6 232 373**

**Description**

La présente invention se rapporte à une composition cosmétique et/ou dermatologique à usage topique contenant de l'acide ascorbique et au moins un polymère ou copolymère de N-vinylimidazole , dans un milieu physiologiquement acceptable comprenant une phase aqueuse.

Il est connu d'introduire dans des compositions cosmétiques divers actifs destinés à apporter des traitements spécifiques à la peau et/ou aux cheveux. Toutefois, certains de ces actifs présentent l'inconvénient d'être instables en milieu aqueux et de se dégrader facilement au contact de l'eau, en particulier à cause de phénomènes d'oxydation. Ils perdent ainsi rapidement leur activité au cours du temps et cette instabilité va à l'encontre de l'efficacité recherchée.

On cherche ainsi depuis longtemps à formuler l'acide ascorbique ou vitamine C, du fait de ses nombreuses propriétés bénéfiques. En particulier, l'acide ascorbique stimule la synthèse du tissu conjonctif et notamment du collagène, renforce les défenses du tissu cutané contre les agressions extérieures telles que les rayonnements ultraviolets et la pollution, compense la déficience en vitamine E de la peau, dépigmente la peau et possède une fonction anti-radicaux libres. Ces deux dernières propriétés en font un excellent candidat comme actif cosmétique ou dermatologique pour lutter contre le vieillissement de la peau ou prévenir celui-ci. Malheureusement, en raison de sa structure chimique (d'alpha-cétolactone), l'acide ascorbique est très sensible à certains paramètres de l'environnement et notamment aux phénomènes d'oxydation. Il s'ensuit donc une dégradation rapide de l'acide ascorbique formulé en présence de ces paramètres, et plus particulièrement en présence d'oxygène, de lumière, d'ions métalliques, en fonction de la température, ou encore dans certaines conditions de pH (Pharm. Acta. Helv., 1969, 44, 611-667 ; STP Pharma, 1985, 4, 281-286).

Plusieurs solutions ont donc été envisagées dans l'art antérieur pour diminuer et/ou retarder la dégradation de l'acide ascorbique.

Il a ainsi été proposé d'utiliser l'acide ascorbique sous forme de dérivé chimique (ascorbyl phosphate de magnésium, ou esters d'acides gras et d'acide ascorbique), mais la biodisponibilité de ces dérivés est très faible (J. Am. Acad. Dermatol., 1996, 34, 29-33).

L'instabilité de l'acide ascorbique vis à vis de l'oxygène a pu être améliorée en utilisant des conditionnements particuliers comme des bi-compartiments sous atmosphère inerte tels que décrits dans le brevet US-5,935,584, ou bien encore par l'utilisation d'émulsions à deux phases dont l'une est constituée d'une poudre sèche contenant l'acide ascorbique et la seconde d'une phase liquide. Le mélange des deux phases doit s'effectuer au moment de l'utilisation (WO98/43598). Ces solutions présentent des inconvénients au niveau du coût et de la complexité des fabrications ainsi que des contraintes importantes au niveau de l'utilisation.

Une autre solution proposée dans l'art antérieur consiste à utiliser des glycols ou des polyols en forte concentration afin de diminuer la solubilité de l'oxygène dans la formulation, protégeant ainsi l'acide ascorbique (WO-96/24325, EP 0 755 674, US-5,981,578). Les polyols peuvent éventuellement être incorporés dans des liposomes comme décrit dans le brevet US-6,020,367. Mais ces solutions présentent l'inconvénient de conduire à des formulations collantes dont la cosméticité est difficile à améliorer. Par ailleurs la présence d'une forte concentration de ces composés peut provoquer des phénomènes d'irritation.

L'acide ascorbique peut également être formulé dans des milieux anhydres tels que les silicones (US-6,194,452) qui sont capables de créer une barrière anhydre autour de l'acide ascorbique. Un inconvénient majeur de telles solutions résulte du manque de fraîcheur à l'application.

Il subsiste donc le besoin d'une composition utilisable notamment dans le domaine cosmétique, dans laquelle un actif hydrophile et instable en milieu oxydant est stabilisé, qui soit confortable lors de l'application, qui ne provoque aucune irritation de la peau après application, et qui soit compatible avec les contraintes d'une mise en oeuvre industrielle de son procédé de fabrication.

Le but de la présente invention est de proposer une composition contenant un actif sensible à l'oxydation, présentant de bonnes propriétés cosmétiques, tant au niveau du toucher qu'au niveau de la tolérance et dont la conservation dans le temps ne demande pas de précautions particulières.

La Demanderesse a découvert, de manière fortuite, que l'utilisation de polymères ou copolymères de N-vinylimidazole non réticulés dans des compositions dont la phase aqueuse renferme un actif sensible à l'oxydation, tel que l'acide ascorbique, permettait d'atteindre le but précité.

Dans l'art antérieur, certains composés possédant une structure imidazole ont été décrits pour leurs propriétés stabilisatrices. Ainsi, dans la demande de brevet EP 0 586 106, plusieurs molécules à base d'imidazole sont utilisées pour stabiliser certains rétinoïdes contre les dégradations chimiques. D'autre part, des émulsifiants polymériques constitués de N-vinylimidazole, d'acrylates d'alkyles et de vinyl-acétates sont décrits dans le brevet US-4,057,622. Ils sont utilisés dans le but de remplacer les émulsifiants connus afin de pallier leurs inconvénients, en particulier au niveau de l'odeur, et pour stabiliser les émulsions eau-dans-huile. Enfin des copolymères de N-vinylimidazole / N-vinylcaprolactam / N-vinylpyrrolidone sont décrits dans le brevet US-6,191,188. Ils entrent dans la fabrication de compositions renforçantes pour les cheveux.

A la connaissance de la demanderesse, des polymères ou copolymères comportant des unités N-vinylimidazoles n'ont jamais été associés à des actifs hydrophiles sensibles aux dégradations par oxydation dans le but d'améliorer leur stabilité en milieu aqueux. Ceci est vrai en particulier dans le cas de l'acide ascorbique.

La demande WO-02/14876 décrit un dispositif pour la préparation de formulations stables contenant un agent peu soluble dans le milieu de dispersion, un additif de formulation, un milieu de dispersion, et optionnellement un solvant. L'exemple 1 décrit une composition obtenue à partir de ce dispositif. Elle comprend un filtre UV (UVINUL T150) et un milieu de dispersion qui comprend différents copolymères notamment à base de vinylimidazole.

La demande EP-1 064 924 décrit la préparation de copolymères contenant des monomères à base de N-vinylimidazole et des exemples de formulations.

Le brevet US-6,232,373 décrit la préparation et l'utilisation de compositions pulvérulentes contenant, entre autres, un copolymère réticulé obtenu à partir de la copolymérisation d'un monomère vinylhétérocyclique (N-vinylimidazole selon l'exemple 2), d'un second monomère, et d'un agent réticulant. L'exemple 5 de ce brevet divulgue l'association du copolymère et d'un extrait de raisin en vue d'éviter l'apparition d'une couleur caractérisant la dégradation de l'extrait de raisin au cours du temps.

La présente invention a donc pour objet une composition à usage topique, contenant, dans un milieu physiologiquement acceptable comprenant une phase aqueuse, de l'acide ascorbique et au moins un polymère ou copolymère de N-vinylimidazole non réticulé, ledit actif et ledit polymère ou copolymère étant tous deux dans la phase aqueuse. Le copolymère est présent en quantité suffisante pour stabiliser l'acide ascorbique.

L'invention concerne également l'utilisation d'un polymère ou copolymère de N-vinylimidazole non réticulé pour stabiliser l'acide ascorbique, en milieu aqueux.

Selon l'invention, par actif hydrophile on entend un composé ayant une solubilité dans l'eau d'au moins 0,25 % à température ambiante (25°C).

Selon l'invention, par actif hydrophile *sensible à l'oxydation* on entend tout actif d'origine naturelle ou synthétique susceptible de subir une dégradation par un mécanisme d'oxydation. Ce phénomène d'oxydation peut avoir plusieurs causes, en particulier la présence d'oxygène, de lumière, d'ions métalliques, une température élevée, ou encore certaines conditions de pH.

Parmi les actifs hydrophiles sensibles à l'oxydation on préfèrera plus particulièrement l'acide ascorbique.

Selon l'invention, par polymère ou copolymère de N-vinylimidazole non réticulé on entend tout polymère comportant des unités N-vinylimidazole, et ne comportant pas d'agent réticulant. Des copolymères convenant à la mise en oeuvre de l'invention sont par exemple les copolymères associant des sous unités N-vinylimidazoles avec des sous unités N-vinylpyrrolidone et/ou N-vinylcaprolactam.

Dans un aspect avantageux de l'invention, le copolymère possède une fraction molaire en unité N-vinylimidazole comprise entre 0,1 et 1, et plus préférentiellement entre 0,4 et 0,9.

Selon un aspect avantageux de l'invention le rapport molaire entre l'équivalent unité N-vinylimidazole et l'actif hydrophile sensible à l'oxydation varie entre 0,004 et 16 et préférentiellement entre 0,01 et 1.

De façon préférentielle on utilisera un copolymère de N-vinylimidazole / N-vinylpyrrolidone.

La masse molaire moyenne en poids des polymères de N-vinylimidazole sera avantageusement comprise entre 1000 et $1 \times 10^7$ et de préférence entre 5000 et $5 \times 10^6$.

On pourra utiliser à cet effet, le copolymère vinylpyrrolidone/vinylimidazole (50/50) ayant une masse molaire moyenne en poids de 1 200 000 vendu sous la référence LUVITEC VPI 55K72W par la société BASF ou le copolymère vinylpyrrolidone/vinylimidazole (50/50) ayant une masse molaire moyenne en poids de 10 000 vendu sous la référence LUVITEC VPI 55K18P par la société BASF. Les polymères ou copolymères selon l'invention peuvent par exemple être préparés selon la méthode décrite dans la demande de brevet WO-97/45517.

Le copolymère est présent dans la composition selon l'invention en quantité suffisante pour obtenir l'effet recherché, c'est à dire en quantité suffisante pour stabiliser l'acide ascorbique. De préférence le copolymère est présent à une concentration comprise entre 0,1 et 5 % en poids, par rapport au poids total de la phase aqueuse, et plus particulièrement à une concentration comprise entre 0,1 et 2 % en poids, par rapport au poids total de la phase aqueuse.

Les compositions utilisées selon l'invention sont destinées à une application topique sur la peau et/ou ses phanères et contiennent donc un milieu physiologiquement acceptable, c'est-à-dire compatible avec les tissus cutanés tels que la peau, le cuir chevelu, les cils, les sourcils, les cheveux, les ongles et les muqueuses. Ce milieu physiologiquement acceptable comprend au moins une phase aqueuse et éventuellement un solvant organique physiologiquement acceptable choisi par exemple parmi les alcools inférieurs comportant de 1 à 8 atomes de carbone et en particulier de 1 à 6 atomes de carbone, comme l'éthanol, l'isopropanol, le propanol, le butanol ; les polyéthylène glycols ayant de 6 à 80 unités oxyde d'éthylène ; les polyols comme le propylène glycol, l'isoprène glycol, le butylène glycol, la glycérine, le sorbitol.

Quand le milieu physiologiquement acceptable est un milieu aqueux, il a généralement un pH compatible avec la peau, allant de préférence de 3 à 9 et mieux de 3,5 à 7,5.

Les compositions selon l'invention peuvent se présenter sous toutes les formes galéniques classiquement utilisées pour une application topique et notamment sous forme de solutions aqueuses, hydroalcooliques, d'émulsions huile-dans-eau (H/E) ou eau-dans-huile (E/H) ou multiple (triple: E/H/E ou H/E/H), de gels aqueux, ou de dispersions d'une phase grasse dans une phase aqueuse à l'aide de sphérules, ces sphérules pouvant être des nanoparticules polymériques telles que les nanosphères et les nanocapsules, ou des vésicules lipidiques de type ionique et/ou non ionique (liposomes, niosomes, oléosomes). Ces compositions sont préparées selon les méthodes usuelles.

En outre, les compositions utilisées selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

Quand la composition utilisée selon l'invention comporte une phase huileuse, celle-ci contient de préférence au moins une huile. Elle peut contenir en outre d'autres corps gras.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité;
- les esters et les éthers de synthèse, notamment d'acides gras, comme les huiles de formules $R^1COOR^2$ et $R^1OR^2$ dans laquelle $R^1$ représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les résines de silicone telles que la trifluorométhyl-C1-4-alkyldiméthicone et la trifluoropropyldiméthicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Selon un mode particulier de réalisation de l'invention, la composition selon l'invention est une émulsion eau-dans-huile (E/H) ou huile-dans-eau (H/E). La proportion de la phase huileuse de l'émulsion peut aller de 5 à 80 % en poids, et de préférence de 5 à 50 % en poids par rapport au poids total de la composition.

Les émulsions contiennent généralement au moins un émulsionnant choisi parmi les émulsionnants amphotères, anioniques, cationiques ou non ioniques, utilisés seuls ou en mélange, et éventuellement un co-émulsionnant. Les émulsionnants sont choisis de manière appropriée suivant l'émulsion à obtenir (E/H ou H/E). L'émulsionnant et le co-émulsionnant sont généralement présents dans la composition, en une proportion allant de 0,3 à 30 % en poids, et de préférence de 0,5 à 20 % en poids par rapport au poids total de la composition.

Pour les émulsions E/H, on peut citer par exemple comme émulsionnants les dimethicone copolyols tels que le mélange de cyclomethicone et de dimethicone copolyol, vendu sous la dénomination « DC 5225 C» par la société Dow Corning, et les alkyl-dimethicone copolyols tels que le Laurylmethicone copolyol vendu sous la dénomination "Dow Corning 5200 Formulation Aid" par la société Dow Corning et le Cetyl dimethicone copolyol vendu sous la dénomination Abil EM 90$^R$ par la société Goldschmidt. On peut aussi utiliser comme tensioactif d'émulsions E/H, un organopolysiloxane solide élastomère réticulé comportant au moins un groupement oxyalkyléné, tel que ceux obtenus selon le mode opératoire des exemples 3, 4 et 8 du document US-A-5,412,004 et des exemples du document US-A-5,811,487, notamment le produit de l'exemple 3 (exemple de synthèse) du brevet US-A-5,412,004. et tel que celui commercialisé sous la référence KSG 21 par la société Shin Etsu. On peut également utiliser comme émulsionnant un oligomère ou un polymère dérivé de polyoléfine à terminaison succinique, celui-ci est de préférence une polyoléfine à terminaison succinique estérifiée ou amidifiée, ou un sel d'une telle polyoléfine, et en particulier du polyisobutylène à terminaison succinique estérifiée ou amidifiée tels que les produits commercialisés sous les dénominations L5603 et L2721 et OS131769 par la société Lubrizol.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose ; et leurs mélanges tels que le mélange de stéarate de glycéryle et de stéarate de PEG-40.

Selon un autre mode de réalisation de l'invention, la composition utilisée contient en outre au moins un actif choisi parmi les agents desquamants capables d'agir, soit en favorisant l'exfoliation, soit sur les enzymes impliquées dans la desquamation ou la dégradation des cornéodesmosomes, les agents hydratants, les agents dépigmentants ou pro-pigmentants, les agents anti-glycation, les inhibiteurs de NO-synthase, les inhibiteurs de 5$\alpha$-réductase, les inhibiteurs de lysyl et/ou prolyl hydroxylase, les agents stimulant la synthèse de macromolécules dermiques ou épidermiques et/ou empêchant leur dégradation, les agents stimulant la prolifération des fibroblastes ou des kératinocytes et/ou la différenciation des kératinocytes, les agents myorelaxants, les agents anti-microbiens, les agents tenseurs, les agents anti-pollution ou anti-radicalaires, les agents anti-inflammatoires, les actifs lipolytiques ou ayant une activité favorable, directe ou indirecte, sur la diminution du tissu adipeux, les agents agissant sur la microcirculation, et les agents agissant sur le métabolisme énergétique des cellules.

De façon connue, la composition cosmétique ou dermatologique de l'invention peut contenir également des adjuvants habituels dans le domaine cosmétique ou dermatologique, tels que les gélifiants hydrophiles ou lipophiles, les conservateurs, les solvants, les parfums, les charges, les filtres UV, les bactéricides, les absorbeurs d'odeur, les matières colorantes, les extraits végétaux, les sels. Les quantités de ces différents adjuvants sont celles classiquement utilisées dans le domaine considéré, et par exemple de 0,01 à 20 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple les pigments, la poudre de silice ; le talc ; les particules de polyamide et notamment celles vendues sous la dénomination ORGASOL par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP ; les poudres expansées telles que les microsphères creuses et notamment, les microsphères commercialisées sous la dénomination EXPANCEL par la société Kemanord Plast ou sous la dénomination MICROPEARL F 80 ED par la société Matsumoto ; les microbilles de résine de silicone telles que celles commercialisées sous la dénomination TOSPEARL par la société Toshiba Silicone ; et leurs mélanges. Ces charges peuvent être présentes dans des quantités allant de 0 à 20 % en poids et de préférence de 1 à 10 % en poids par rapport au poids total de la composition.

Selon un mode préféré de réalisation, les compositions conformes à l'invention peuvent comporter en plus au moins un agent photoprotecteur organique et/ou au moins un agent photoprotecteur inorganique actif dans l'UVA et/ou l'UVB (absorbeurs), hydrosolubles ou liposolubles ou bien insolubles dans les solvants cosmétiques couramment utilisés.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans les demandes US5,237,071, US5,166,355, GB2303549, DE 197 26 184 et EP893119 ; les polymères filtres et silicones filtres tels que ceux décrits notamment dans la demande WO-93/04665 ; les dimères dérivés d'α-alkylstyrène tels que ceux décrits dans la demande de brevet DE19855649 ; les 4,4-diarylbutadiènes tels que décrits dans les demandes EP0967200, DE19746654, DE19755649, EP-A-1008586, EP1133980 et EP133981 et leurs mélanges.

A titre d'illustration, comme d'agents photoprotecteurs actifs dans l'UV-A et/ou l'UV-B, on peut citer, désignés ci-dessous sous leur nom INCI :

les dérivés de l'acide p-aminobenzoïque (PABA), en particulier le PABA, l'éthyl PABA, l'éthyl Dihydroxypropyl PABA, l'éthylhexyl Diméthyl PABA (vendu notamment sous le nom « ESCALOL 507 » par ISP), le glyceryl PABA, ou le PEG-25 PABA (vendu sous le nom « UVINUL P25 » par BASF),

les dérivés salicyliques, en particulier l'homosalate (vendu sous le nom « EUSOLEX HMS » par RONA/EM INDUSTRIES), l'éthylhexyl salicylate (vendu sous le nom « NEO HELIOPAN OS» par HAARMANN et REIMER), le dipropyleneglycol salicylate (vendu sous le nom « DIPSAL » par SCHER), ou le TEA salicylate (vendu sous le nom « NEO HELIOPAN TS » par HAARMANN et REIMER),

les dérivés de dibenzoylméthane, en particulier le butyl Methoxydibenzoylmethane (vendu notamment sous le nom commercial « PARSOL 1789 » par HOFFMANN LA ROCHE), ou l'isopropyl Dibenzoylmethane,

les dérivés cinnamiques, en particulier l'éthylhexyl Methoxycinnamate (vendu notamment sous le nom commercial « PARSOL MCX » par HOFFMANN LA ROCHE), l'isopropyl methoxy cinnamate, l'isoamyl Methoxy cinnamate (vendu sous le nom commercial «NEO HELIOPAN E 1000» par HAARMANN et REIMER), le cinoxate, le DEA methoxycinnamate, le diisopropyl methyl cinnamate, ou le glyceryl ethylhexanoate dimethoxycinnamate,

les dérivés de β,β-diphénylacrylate, en particulier l'octocrylene (vendu notamment sous le nom commercial « UVINUL N539 » par BASF), ou l'étocrylene, (vendu notamment sous le nom commercial « UVINUL N35 » par BASF),

les dérivés de la benzophénone, en particulier la benzophenone-1 (vendue sous le nom commercial « UVINUL 400 » par BASF), la benzophenone-2 (vendue sous le nom commercial « UVINUL D50 » par BASF), la benzophenone-3 ou oxybenzone (vendue sous le nom commercial « UVINUL M40 » par BASF), la benzophenone-4 (vendue sous le nom commercial « UVINUL MS40 » par BASF), la benzophenone-5, la benzophenone-6 (vendue sous le nom commercial « HELISORB 11 » par NORQUAY), la benzophenone-8 (vendue sous le nom commercial « SPECTRA-SORB UV-24 » PAR AMERICAN CYANAMID), la benzophenone-9 (vendue sous le nom commercial « UVINUL DS-49» par BASF), la benzophenone-12, ou le 2-(4-diéthylamino-2-hydroxybenzoyl)-benzoate de n-hexyle,

les dérivés du benzylidène camphre, en particulier le 3-benzylidene camphor (fabriqué sous le nom « MEXORYL SD» par CHIMEX), le 4-methylbenzylidene camphor (vendu sous le nom « EUSOLEX 6300 » par MERCK), le benzylidene camphor Sulfonic Acid (fabriqué sous le nom « MEXORYL SL» par CHIMEX), le camphor benzalkonium methosulfate (fabriqué sous le nom « MEXORYL SO » par CHIMEX), le terephthalylidene dicamphor sulfonic acid (fabriqué sous le nom « MEXORYL SX » par CHIMEX), ou le polyacrylamidomethyl benzylidene camphor (fabriqué sous le nom « MESORYL SW » par CHIMEX),

les dérivés de benzimidazole, en particulier le phenylbenzimidazole sulfonic acid (vendu notamment sous le nom commercial « EUSOLEX 232 » par MERCK), ou le disodium phenyl dibenzimidazole tetra-sulfonate (vendu sous le nom commercial « NEO HELIOPAN AP » par HAARMANN et REIMER),

les dérivés de triazine, en particulier l'anisotriazine (vendue sous le nom commercial «TINOSORB S » par CIBA SPECIALTY CHEMICALS), l'éthylhexyl triazone (vendue notamment sous le nom commercial «UVINUL T150 » par BASF), la diethylhexyl butamido triazone (vendue sous le nom commercial « UVASORB HEB » par SIGMA 3V), ou la 2,4,6- tris-(4'amino-benzalmalonate de diisobutyle)-s-triazine,

les dérivés de benzotriazole, en particulier, le drometrizole trisiloxane (vendu sous le nom « SILATRIZOLE » par

RHODIA CHIMIE), le méthylène bis-benzotriazolyl tetramethylbutylphénol (vendu sous forme solide sous le nom commercial « MIXXIM BB/100 » par FAIRMOUNT CHEMICAL ou sous forme micronisé en dispersion aqueuse sous le nom commercial « TINOSORB M » par CIBA SPECIALTY CHEMICALS),

les dérivés anthraniliques, en particulier le menthyl anthranilate (vendu sous le nom commercial « NEO HELIOPAN MA » par HAARMANN et REIMER),

les dérivés d'imidazolines, en particulier l'éthylhexyl dimethoxybenzylidene dioxoimidazoline propionate,

les dérivés de benzalmalonate, en particulier le polyorganosiloxane à fonctions benzalmalonate (vendu sous la dénomination commerciale « PARSOL SLX » par HOFFMANN LA ROCHE),

les dérivés de 4,4-diarylbutadiène, en particulier le 1,1'-dicarboxy (2,2'-diméthyl-propyl)-4,4-diphénylbutadiène,

et leurs mélanges.

Les agents photoprotecteurs organiques plus particulièrement préférés sont choisis parmi l'éthylhexyl salicylate, l'éthylhexyl methoxycinnamate, l'octocrylene, le phenylbenzimidazole sulfonic acid, la benzophenone-3, la benzophenone-4, la benzophenone-5, la 4-methylbenzylidene camphor, le terephthalylidene dicamphor sulfonic acid, le disodium phenyl dibenzimidazole tetra-sulfonate, la 2,4,6-tris-(4'-amino benzalmalonate de diisobutyle)-s-triazine, l'anisotriazine, l'éthylhexyl triazone, la diethylhexyl butamido triazone, le methylène bis-benzotriazolyl tetramethylbutylphénol, le drometrizole trisiloxane, le 1,1'-dicarboxy (2,2'-diméthyl-propyl)-4,4-diphénylbutadiène, et leurs mélanges.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans les compositions selon l'invention dans des proportions allant de 0,1 à 20% en poids par rapport au poids total de la composition, et de préférence allant de 0,2 à 15% en poids par rapport au poids total de la composition.

La composition selon l'invention peut être appliquée sur la peau, les poils, les cils, les cheveux, les ongles ou les lèvres, suivant l'usage auquel elle est destinée. Elle peut ainsi être utilisée dans un procédé de traitement cosmétique de la peau, comprenant l'application de la composition selon l'invention sur la peau.

En variante, la composition selon l'invention peut être utilisée pour la fabrication d'une préparation dermatologique.

Les exemples qui suivent servent à illustrer l'invention sans toutefois présenter un caractère limitatif. Les composés sont, selon le cas, cités en noms chimiques ou en noms CTFA (International Cosmetic Ingredient Dictionary and Handbook).

## EXEMPLES

### Exemple 1 : Test de conservation accéléré.

Ce test a pour but d'étudier la dégradation d'un actif hydrophile sensible à l'oxydation après deux mois de conservation à 45°C. Diverses solutions ont été réalisées, et leurs compositions sont regroupées dans le tableau suivant :

Tableau I :

| Compositions (dans l'eau) | solution A (Témoin) | solution B | solution C | solution D |
|---|---|---|---|---|
| acide ascorbique | 15% | 15% | 15% | 15% |
| polymère 1 | - | 1% | - | - |
| polymère 2 | - | - | 1% | - |
| polymère 3 | - | - | - | 1% |

Toutes les solutions sont ramenées à pH 6 avec KOH 8,9 mole/l

Les pourcentages des polymères sont donnés en matière active.

Polymère 1 : Copolymère vinylpyrrolidone-vinylimidazole (50/50) vendu sous la référence LUVITEC VPI 55K72W de la société BASF (Masse mol. moyenne en poids $1,2.10^6$).

Polymère 2 : Copolymère vinylpyrrolidone-vinylimidazole (50/50) vendu sous la référence LUVITEC VPI 55K18P de la société BASF (Masse mol. moyenne en poids 10000).

Polymère 3 : Polyvinylpyrrolidone vendu sous la référence KOLLIDON 12PF de la société BASF (Masse mol. Moyenne en poids 3000).

Le taux de dégradation mesuré est donné par le rapport :

$$(C_0 - C_{2mois})/C_0$$

avec $C_0$ concentration en acide ascorbique à t=0 et $C_{2mois}$ la concentration en acide ascorbique à t=2 mois, dans les conditions indiquées dans le tableau ci-dessus.

La concentration en acide ascorbique est déterminée par la technique HPLC (système LaChrom Merck). Les conditions analytiques sont les suivantes :

Colonne Lichrosphere100 RP18 (250 mm)
Eluant : tampon phosphate O,1 M, pH 2,1
Débit : 1 ml/min.

Détection à 257 nm

Dilution de l'échantillon tel que la concentrattion en acide ascorbique soit comprise entre 0,05 et 1 mg/ml.

Les résultats obtenus sont regroupés dans le tableau II suivant :

Tableau II :

|  | Taux de dégradation après 2 mois à 45°C (en %) | |
|---|---|---|
|  | sous air, flacon verre ambré | sous azote, flacon aluminium |
| **solution A** | 43 | 19,4 |
| **solution B** | 10,8 | 1 |
| **solution C** | 23,4 | 4,5 |
| **solution D** | 35,8 | 15,7 |

On constate d'après le tableau II que la stabilité de l'acide ascorbique est améliorée en présence des polymères 1 et 2 de l'invention, même en présence de l'oxygène de l'air, comparativement au témoin. On constate également que l'homopolymère de N-vinylpyrrolidone seul ne suffit pas à stabiliser de façon efficace la solution d'acide ascorbique.

Les polymères cités étant hydrophiles, il suffira de les ajouter à une solution aqueuse d'acide ascorbique pour stabiliser ce dernier.

**Exemple 2 : Emulsion fluide E/H**

On prépare la composition suivante de manière classique pour l'homme du métier.

**Phase A :**

| Cetyl dimethicone copolyol | 1,5 g |
|---|---|
| Polyglycéryl-4 isostéarate | 0,5 g |
| Squalane | 3,7 g |
| Isohexadécane | 7,95 g |
| Polydiméthylsiloxane | 4 g |
| Huile d'abricot | 2,25 g |

**Phase B :**

| | |
|---|---|
| Acide ascorbique | 5 g |
| Hydroxyde de potassium à 50% | 3 g |
| Copolymère vinylpyrrolidone/vinylimidazole (Luvitec VP155K72W de BASF) | 3,3 g |
| Glycérine | 5 g |
| Conservateurs | 0,4 g |
| Eau | 60,4g |
| Poudre de Nylon-12 | 3 g |

On obtient un fluide, doux à l'application avec une bonne stabilité de l'acide ascorbique.

**Exemple 3 : Emulsion E/H fluide pour l'éclat du teint**

On prépare la composition suivante de manière classique pour l'homme du métier.

**Phase A :**

| | |
|---|---|
| Ethanoldiethonium polyisobutenyl triéthylamino-succinate (et) diéthyl éthanolamine (Lubrizol LZ 5603) | 2 g |
| Isohexadécane | 8 g |
| Polyisobutylène hydrogéné | 3,7 g |
| Huile d'abricot | 6,4 g |

**Phase B :**

| | |
|---|---|
| Acide ascorbique | 5 g |
| Hydroxyde de potassium à 50% | 3 g |
| Copolymère vinylpyrrolidone/vinylimidazole, (Luvitec VP155K72W de BASF) | 3,3 g |
| Glycérine | 5 g |
| Conservateurs | 0,4 g |
| Eau | 61,2 g |
| Poudre de Nylon-12 | 2 g |

On obtient une crème de soin pour la peau, douce à l'application avec une bonne stabilité de l'acide ascorbique.

**Exemple 4 : Emulsion fluide, E/H**

On prépare la composition suivante de manière classique pour l'homme du métier.

**Phase A :**

| | |
|---|---|
| Isostéaryl diglycéryl succinate | 4 g |
| Octyl dodecanol | 4g |
| Huile d'abricot | 8 g |
| Cyclométhicone | 12g |
| Céramide de synthèse | 0,1g |
| Gomme de silicone | 6 g |

**Phase B :**

| | |
|---|---|
| Acide ascorbique | 5 g. |
| Hydroxyde de potassium à 50% | 3,07 g |
| Copolymère vinylpyrrolidone/vinylimidazole (Luvitec VP155K72W de BASF) | 1 g |
| Glycérine | 2 g |
| Aluminium starch octenylsuccinate | 0,3 g |

(suite)

**Phase B :**

| | |
|---|---|
| Conservateurs | 0,5 g |
| Eau déminéralisée | 54,03 g |

On obtient une crème de soin pour la peau souple et douce à l'application avec une bonne stabilité de l'acide ascorbique.

## Exemple 5 : Crème visage H/E

On prépare la composition suivante de manière classique pour l'homme du métier.

**Phase A :**

| | |
|---|---|
| Glyceryl stearate et PEG-100 stearate | 2,1 g |
| Polysorbate 60 | 0,9 g |
| Alcool cétylique | 2,6 g |
| Hydrogenated polyisobutene | 12 g |
| Cyclométhicone | 8 g |

**Phase B :**

| | |
|---|---|
| Eau déminéralisée | 59,23 g |
| Glycérine | 2 g |
| Acide ascorbique | 5 g |
| Hydroxyde de potassium à 50% | 3,07 g |
| Copolymère vinylpyrrolidone/vinylimidazole, (Luvitec VP155K72W de BASF) | 1 g |
| Gomme de Xanthane | 0,1 g |
| Carbomer | 0,4 g |

**Phase C :**

| | |
|---|---|
| Triéthanolamine | 0,3 g |
| Eau déminéralisée | 3 g |
| Conservateur | 0,3 g |

On obtient une crème de soin pour la peau riche et douce à l'application, avec une bonne stabilité de l'acide ascorbique.

## Exemple 6 : Emulsion multiple E/H/E

On prépare la composition suivante de manière classique pour l'homme du métier.

Emulsion primaire

**Phase A :**

| | |
|---|---|
| Polyglyceryl-4 isostearate et Cetyldimethicone copolyol et hexyl laurate | 3,5 g |
| Cyclopentasiloxane | 16,5 g |
| Dimethicone (gomme silicone) | 4 g |

**Phase B :**

| | |
|---|---|
| Eau déminéralisée | 46,27 g |
| Acide ascorbique | 15g |
| Hydroxyde de potassium à 50% | 9,33 g |
| Copolymère vinylpyrrolidone/vinylimidazole (Luvitec VP155K72W de BASF) | 3,3 g |
| Glycérine | 2 g |
| Pentasodium ethylenediamine tetramethylene phosphonate (solution aqueuse à 33%) | 0,1 g |

Emulsion Multiple

**Phase A**

| | |
|---|---|
| Emulsion primaire | 20 g |
| Cyclopentasiloxane | 5 g |
| Huile d'amande d'abricot | 5 g |

**Phase B :**

| | |
|---|---|
| Ammonium polyacryloyldimethyl taurate | 0,4 g |
| Acrylates/C 10-30 alkyl acrylate crosspolymer | 0,6 g |
| Eau déminéralisée | 35,7 g |
| Conservateur | 1 g |

**Phase C :**

| | |
|---|---|
| Eau déminéralisée | 5,7 g |
| Triethanolamine | 0,7 g |

**Phase D :**

| | |
|---|---|
| Ammonium polyacryloyldimethyl taurate | 0,6 g |
| Eau déminéralisée | 25,3 g |

On obtient une crème blanche et fraîche conférant une bonne stabilité à l'acide ascorbique et apte à être appliquée sur la peau.

**Exemple 7 : Microcapsules d'acide ascorbique**

On prépare une solution aqueuse d'acide ascorbique à 15% en poids, à pH 6, contenant 3,3g de copolymère vinylpyrrolidone/vinylimidazole (Luvitec VPI55K72W® de BASF). On émulsionne 5ml de cette solution dans 50 ml de chlorure de méthylène contenant 5% d'acéto-propionate de cellulose (CAP-482-0.5®, Eastman Chemical) à l'aide d'un homogénéiseur de type rotor-stator, pendant 5 min en maintenant la température en dessous de 25°C. Cette émulsion primaire est ensuite dispersée dans 500 ml d'une solution aqueuse contenant 1% d'alcool polyvinylique (Airvol 203®, AirProducts) et 7% de chlorure de sodium, à l'aide d'un disperseur Moritz pendant 20 min à température ambiante.
Le solvant de la suspension est ensuite évaporé à l'aide d'un évaporateur rotatif (Büchi B-480) pendant 5 heures à 40°C, à une pression de 75 kPa.
On obtient une dispersion de microcapsules dont la taille moyenne est de 20 $\mu$m avec un taux d'encapsulation de 85%, et un rendement de fabrication de 100%.

**Exemple 8 :Crème de jour contenant les microcapsules selon l'Exemple** 7

**Phase A**

| | |
|---|---|
| Alcool cétylique | 4 g |
| Tristéarate de sorbitane | 0,9 g |
| Stéarate de polyéthylèneglycol | 2 g |
| Stéarate de glycérol | 3 g |
| Myristate de myristyle | 2 g |
| Palmitate d'octyle | 4,5 g |
| Parsol MCX ® (vendu par Hoffman-Laroche) | 3 g |
| Cyclopentasiloxane | 5 g |
| Conservateur | 0,1 g |

**Phase B**

| | |
|---|---|
| Eau déminéralisée | 60,3 g |
| Conservateur | 0,15g |
| Séquestrant | 0,05g |

**Phase C**

| | |
|---|---|
| Poudre de microcapsules selon l'exemple 7 | 15g |

**Exemple 9 : Crème E/H**

On prépare la composition suivante de manière classique pour l'homme du métier.

| | |
|---|---|
| Eau | 21,05 g |
| Phenoxyethanol | 0,5 g |
| Methylparaben | 0,3 g |
| Water | 71,3 g |
| Kojic acid | 1 g |
| Vinylpyrrolidone/vinylimidazole copolymer | 0,5 g |
| Biosaccharide Gum-1 | 2 g |
| Polysorbate 20 | 1 g |
| Cyclopentasiloxane (and) dimethicone copolyol | 10 g |
| Cyclopentasiloxane | 8 g |
| Dimethicone (and) dimethicone/vinyl dimethicone crosspolymer | 3 g |
| Tocopherol (and) glycine soja (soybean) oil | 0,4 g |
| Polyacrylamide (and) C13-14 isoparaffin (and) laureth-7 | 2 g |

On obtient une crème de soin pour la peau, avec une bonne stabilité de l'acide ascorbique

**Exemple 10 : Gel E/H**

On prépare la composition suivante de manière classique pour l'homme du métier.

| | |
|---|---|
| Cyclopentasiloxane (and) dimethicone copolyol | 17,5 g |
| Sodium methylparaben | 0,3 g |
| Chlorphenesin | 0,25 g |
| Eau | 81,2g |
| Phloroglucinol | 0,5 g |
| Vinylpyrrolidone/vinylimidazole copolymer | 0,25 g |

On obtient un gel pour la peau, avec une bonne stabilité de l'acide ascorbique

## Revendications

1. Composition à usage topique contenant, dans un milieu physiologiquement acceptable comprenant un phase aqueuse, de l'acide ascorbique et au moins un polymère ou copolymère de N-vinylimidazole non réticulé, l'acide ascorbique et ledit polymère ou copolymère étant tous deux dans la phase aqueuse.

2. Composition selon la revendication précédente **caractérisée en ce que** le copolymère non réticulé est une association de sous unités N-vinylimidazoles avec des sous unités N-vinylpyrrolidone et/ou N-vinylcaprolactam.

3. Composition selon la revendication 1 ou 2 **caractérisée en ce que** le copolymère non réticulé est un copolymère de N-vinylimidazole / N-vinylpyrrolidone.

4. Composition selon l'une quelconque des revendications 1 à 3 **caractérisée en ce que** le copolymère non réticulé est choisi parmi un copolymère vinylpyrrolidone/vinylimidazole (50/50) ayant une masse molaire moyenne en poids de 1 200 000 et un copolymère vinylpyrrolidone/vinylimidazole (50/50) ayant une masse molaire moyenne en poids de 10 000.

5. Composition selon l'une quelconque des revendications 1 à 4 **caractérisée en ce que** le rapport molaire entre l'équivalent unité N-vinylimidazole et l'acide ascorbique varie entre 0,004 et 16.

6. Composition selon la revendication précédente **caractérisée en ce que** le rapport molaire entre l'équivalent unité N-vinylimidazole et l'acide ascorbique varie entre 0,01 et 1.

7. Composition selon l'une quelconque des revendications précédentes **caractérisée en ce que** le polymère ou copolymère est présent à une concentration comprise entre 0,1 et 5 % en poids de la phase aqueuse.

8. Composition selon la revendication précédente **caractérisée en ce que** le polymère ou Copolymère est présent à une concentration comprise entre 0,1 et 2 % en poids de la phase aqueuse.

9. Composition selon l'une quelconque des revendications précédentes **caractérisée en ce que** le polymère ou copolymère possède une fraction molaire en unité N-vinyllmidazole comprise entre 0,1 et 1.

10. Composition selon la revendication précédente **caractérisée en ce que** le polymère ou copolymère possède une fraction molaire en unité N-vinylimidazole comprise entre 0,4 et 0,9.

11. Utilisation d'un polymère ou copolymère de N-vinylimidazole non réticulé pour stabiliser l'acide ascorbique en milieu aqueux.

12. Utilisation selon la revendication 13 **caractérisée en ce que** le copolymère non réticulé est une association des sous unités N-vinylimidazole, N-vinylpyrrolidone, et/ou N-vinylcaprolactam.

13. Utilisation selon la revendication précédente **caractérisée en ce que** le copolymère non réticulé est un copolymère de N-vinylimidazole / N-vinylpyrrolidone.

14. Utilisation selon la revendication 12 **caractérisée en ce que** le copolymère non réticulé est choisi parmi un copolymère vinylpyrrolidone/vinylimidazole (50/50) ayant une masse molaire moyenne en poids de 1 200 000 et un copolymère vinylpyrrolidone/vinylimidazole (50/50) ayant une masse molaire moyenne en poids de 10 000.

15. Procédé de traitement cosmétique de la peau, des poils, des cils, des cheveux, des ongles ou des lèvres comprenant l'application sur la peau, les poils, les cils, les cheveux, les ongles ou les lèvres d'une composition selon l'une quelconque des revendications 1 à 10.

**Claims**

1. Composition for topical use comprising, in a physiologically acceptable medium comprising an aqueous phase, ascorbic acid and at least one non-crosslinked N-vinylimidazole polymer or copolymer, the ascorbic acid and the said polymer or copolymer both being in the aqueous phase.

2. Composition according to the preceding claim, **characterized in that** the non-crosslinked copolymer is a combination of N-vinylimidazole subunits with N-vinylpyrrolidone and/or N-vinylcaprolactam subunits.

3. Composition according to Claim 1 or 2, **characterized in that** the non-crosslinked copolymer is an N-vinylimidazole/N-vinylpyrrolidone copolymer.

4. Composition according to any one of Claims 1 to 3, **characterized in that** the non-crosslinked copolymer is chosen from a vinylpyrrolidone/vinylimidazole (50/50) copolymer having a weight-average molar mass of 1 200 000 and a vinylpyrrolidone/vinylimidazole (50/50) copolymer having a weight-average molar mass of 10 000.

5. Composition according to any one of Claims 1 to 4, **characterized in that** the molar ratio of the N-vinylimidazole unit equivalent to the ascorbic acid varies between 0.004 and 16.

6. Composition according to the preceding claim, **characterized in that** the molar ratio of the N-vinylimidazole unit equivalent to the ascorbic acid varies between 0.01 and 1.

7. Composition according to any one of the preceding claims, **characterized in that** the polymer or copolymer is present at a concentration of between 0.1 and 5% by weight of the aqueous phase.

8. Composition according to the preceding claim, **characterized in that** the polymer or copolymer is present at a concentration of between 0.1 and 2% by weight of the aqueous phase.

9. Composition according to any one of the preceding claims, **characterized in that** the polymer or copolymer has a molar fraction of N-vinylimidazole units of between 0.1 and 1.

10. Composition according to the preceding claim, **characterized in that** the polymer or copolymer has a molar fraction of N-vinylimidazole units of between 0.4 and 0.9.

11. Use of a non-crosslinked N-vinylimidazole polymer or copolymer to stabilize ascorbic acid in an aqueous medium.

12. Use according to Claim 13, **characterized in that** the non-crosslinked copolymer is a combination of the N-vinylimidazole, N-vinylpyrrolidone and/or N-vinylcaprolactam subunits.

13. Use according to the preceding claim, **characterized in that** the non-crosslinked copolymer is an N-vinylimidazole/N-vinylpyrrolidone copolymer.

14. Use according to Claim 12, **characterized in that** the non-crosslinked copolymer is chosen from a vinylpyrrolidone/vinylimidazole (50/50) copolymer having a weight-average molar mass of 1 200 000 and a vinylpyrrolidone/vinylimidazole (50/50) copolymer having weight-average molar mass of 10 000.

15. Method for the cosmetic treatment of the skin, body hairs, eyelashes, hair, nails or lips, comprising the application, to the skin, body hairs, eyelashes, hair, nails or lips, of a composition according to any one of Claims 1 to 10.

**Patentansprüche**

1. Zusammensetzung zur topischen Anwendung, die in einem physiologisch akzeptablen Medium, das eine wässrige Phase aufweist, Ascorbinsäure und mindestens ein nicht vernetztes Polymer oder Copolymer von N-Vinylimidazol enthält, wobei die Ascorbinsäure und das Polymer oder Copolymer beide in der wässrigen Phase enthalten sind.

2. Zusammensetzung nach dem vorhergehenden Anspruch, **dadurch gekennzeichnet, dass** das nicht vernetzte Copolymer eine Kombination von N-Vinylimidazoluntereinheiten mit N-Vinylpyrrolidon- und/oder N-Vinylcaprolactamuntereinheiten ist.

3. Zusammensetzung nach Anspruch 1 oder 2, **dadurch gekennzeichnet, dass** das nicht vernetzte Copolymer ein N-Vinylimidazol/N-Vinylpyrrolidon-Copolymer ist.

4. Zusammensetzung nach einem der Ansprüche 1 bis 3, **dadurch gekennzeichnet, dass** das nicht vernetzte Copolymer unter einem Copolymer Vinylpyrrolidon/Vinylimidazol (50/50) mit einer gewichtsmittleren Molmasse von 1 200 000 und einem Copolymer Vinylpyrrolidon/Vinylimidazol (50/50) mit einer gewichtsmittleren Molmasse von 10 000 ausgewählt ist.

5. Zusammensetzung nach einem der Ansprüche 1 bis 4, **dadurch gekennzeichnet, dass** das Molverhältnis eines Äquivalents N-Vinylimidazoleinheit und der ascorbinsäure im Bereich von 0,004 bis 16 liegt.

6. Zusammensetzung nach dem vorhergehenden Anspruch, **dadurch gekennzeichnet, dass** das Molverhältnis eines Äquivalents N-Vinylimidazoleinheit und der Ascorbinsäure im Bereich von 0,01 bis 1 liegt.

7. Zusammensetzung nach einem der vorhergehenden Ansprüche, **dadurch gekennzeichnet, dass** das Polymer oder Copolymer in einer Konzentration von 0,1 bis 5 % des Gewichts der wässrigen Phase vorliegt.

8. Zusammensetzung nach dem vorhergehenden Anspruch, **dadurch gekennzeichnet, dass** das Polymer oder Copolymer in einer Konzentration von 0,1 bis 2 % des Gewichts der wässrigen Phase vorliegt.

9. Zusammensetzung nach einem der vorhergehenden Ansprüche, **dadurch gekennzeichnet, dass** das Polymer oder Copolymer einen molaren Anteil der N-Vinylimidazoleinheit von 0,1 bis 1 aufweist.

10. Zusammensetzung nach dem vorhergehenden Anspruch, **dadurch gekennzeichnet, dass** das Polymer oder Copolymer einen molaren Anteil der N-Vinylimidazoleinheit von 0,4 bis 0,9 aufweist.

11. Verwendung eines nicht vernetzten Polymers oder Copolymers von N-Vinylimidazol zur Stabilisierung von Ascorbinsäure in einem wässrigen Medium.

12. Verwendung nach Anspruch 13, **dadurch gekennzeichnet, dass** das nicht vernetzte Copolymer eine Kombination der Untereinheiten N-Vinylimidazol, N-Vinylpyrrolidon und/oder N-Vinylcaprolactam ist.

13. Verwendung nach dem vorhergehenden Anspruch, **dadurch gekennzeichnet, dass** das nicht vernetzte Copolymer ein N-Vinylimidazol/ N-Vinylpyrrolidon-Copolymer ist.

14. Verwendung nach Anspruch 12, **dadurch gekennzeichnet, dass** das nicht vernetzte Copolymer unter einem Copolymer Vinylpyrrolidon/Vinylimidazol (50/50) mit einer gewichtsmittleren Molmasse von 1 200 000 und einem Copolymer Vinylpyrrolidon/Vinylimidazol (50/50) mit einer gewichtsmittleren Molmasse von 10 000 ausgewählt ist.

15. Verfahren zur kosmetischen Behandlung der Haut, der Körperhaare, der Wimpern, der Haare, der Nägel oder der Lippen, das das Auftragen einer Zusammensetzung nach einem der Ansprüche 1 bis 10 auf die Haut, die Körperhaare, die Wimpern, die Haare, die Nägel oder die Lippen umfasst.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5935584 A
- WO 9843598 A
- WO 9624325 A
- EP 0755674 A
- US 5981578 A
- US 6020367 A
- US 6194452 B
- EP 0586106 A
- US 4057622 A
- US 6191188 B
- WO 0214876 A
- EP 1064924 A
- US 6232373 B
- WO 9745517 A
- JP 2295912 A
- US 5412004 A
- US 5811487 A
- US 4367390 A
- EP 863145 A
- EP 517104 A
- EP 570838 A
- EP 796851 A
- EP 775698 A
- EP 878469 A
- EP 933376 A
- EP 507691 A
- EP 507692 A
- EP 790243 A
- EP 944624 A
- EP 669323 A
- US 2463264 A
- US 5237071 A
- US 5166355 A
- GB 2303549 A
- DE 19726184
- EP 893119 A
- WO 9304665 A
- DE 19855649
- EP 0967200 A
- DE 19746654
- DE 19755649
- EP 1008586 A
- EP 1133980 A
- EP 133981 A
- EP 518772 A
- EP 518773 A

**Littérature non-brevet citée dans la description**

- *Pharm. Acta. Helv,* 1969, vol. 44, 611-667
- *STP Pharma,* 1985, vol. 4, 281-286
- *J. Am. Acad. Dermatol.,* 1996, vol. 34, 29-33